# EUROPEAN PATENT APPLICATION

(11) **EP 1 228 707 A1**
(43) Date of publication of application: **07.08.2002**
(21) Application number: 01200365.3
(22) Date of filing: 01.02.2001
(51) Int. Cl.: A23L 1/305, A23J 1/20, A23C 9/15, A23C 9/13, A23C 19/09, A61K 38/16, A61K 38/38

(54) **Use of alpha-lactalbumin as prebiotic agent**

(71) Applicant: CAMPINA MELKUNIE B.V., NL-5301 LB Zaltbommel (NL)
(72) Inventor: Maase, Kamiel, 6702 AB Wageningen (NL); Steijns, Johannes Maria Jozef Margaretha, 5427 SN Boekel (NL)
(74) Representative: Wittop Koning, Tom Hugo

(57) **Abstract**

Described is the use of α-lactalbumin as a prebiotic food, food additive or food supplement as well as dairy products comprising additional α-lactalbumin in an effective bibliotic amount. Further, a pharmaceutical composition for the treatment of gastro-enteritis comprising α-lactalbumin as active compound is described.

## Description

The present invention relates to the use of α-lactalbumin and of α-lactalbumin enriched whey protein concentrate as a prebiotic food, food additive or food supplement.

In the food industry, there is need for food products that may confer health benefits to the consumer by improving the beneficial intestinal microbial population, usually mainly consisting of the lactic acid producing bacteria of the lactobacilli and bifidobacteria.

Two approaches for the preparation of such products are known in the art. The direct approach provides living preparations of the organisms in the food product, usually in fermented dairy products, such as yoghurt. Such beneficial organisms, present in food products are also known as "probiotics". Nutritional preparations comprising probiotics are e.g. described in EP-A-904 784 and in WO-A-99/02170. One of the problems encountered with probiotics is that a substantial proportion thereof does not survive the passage of the gastro-intestinal tract upstream of the colon, the target location for such bacteria.

The above-mentioned problem is avoided by the so-called indirect approach, wherein food supplements are added to the food products, which food supplements support and stimulate the growth of the above-mentioned beneficial organisms resident in the colon. These food additives are known as "prebiotics". A wide range of non-digestible carbohydrates having prebiotic properties have been described. For example the use of fructo-oligosaccharides as prebiotic in natural dessert cream is described in EP-A-970 618; the use of D-tagatose as prebiotic is known from WO-A-9 943 217; inulin as prebiotic in a food preparation is described in WO-A-9 907 239, and β-glucans and derived compounds as prebiotics are described in WO-A-9 826 787. Further, fructan as prebiotic is known from US-5,840,361.

It has now surprisingly been shown that in addition to the above-mentioned carbohydrates, the protein α-lactalbumin, a protein occurring in e.g. bovine milk, shows as significant prebiotic effect. Although it is known that whey can stimulate the growth of bifidobacteria in the colon (see e.g. B.A. Wharton et al. In: Lactoferrin: Structure and Function, T.W. Hutchnes et al (eds.), Plenum Press, New York, USA 1994; S.E. Balmer et al, Arch. Dis. Child. 1989: 1678-1684; B. Kleessen et al, Acta Peadiatr. 1995; 84: 1347-1356), the prebiotic effect thereof has been ascribed to the whey protein glycomacropeptide (GMP), a casein fragment being formed by the rennet in the curdling of milk in the cheese preparation process. It has now been shown that α-lactalbumin and α-lactalbumin enriched whey protein concentrate have improved prebiotic properties compared to whole whey. The invention therefore relates to the novel use of α-lactalbumin and α-lactalbumin enriched whey protein concentrate as a prebiotic food, food ingredient, food additive or food supplement. According to the invention, α-Lactalbumin may be comprised in any food product; however dairy products, especially milk and yoghurt are preferred. With "dairy products" all products derived from milk are meant. However, dairy products that are consumer products, such as milk, cheese, yoghurt, cream etc. are preferred. Industrial materials such as casein and whey protein concentrate are as such not regarded as consumer products. α-Lactalbumin can also be incorporated in a formulation of a dry powder to be dissolved in water or another beverage for the preparation of instant drinks.

Dairy products usually comprise both casein and α-lactalbumin. However, the ratio of α-lactalbumin: casein in dairy products is 0,07 or less.

Therefore, the present invention relates in a second aspect to a food product, preferably a dairy product, enriched in α-lactalbumin, and such a product comprises α-lactalbumin and casein, wherein the weight ratio α-lactalbumin:casein is at least 0,1. This means that on weight basis, the dairy product has a casein content that is at most 10 times as much as the α-lactalbumin content. Thus, a dairy product having such a casein:α-lactalbumin ratio is enriched in α-lactalbumin. Because of the elevated α-lactalbumin level in the dairy product, the dairy product shows a significant and improved prebiotic effect.

Preferably, the α-lactalbumin:casein ratio is 0,1-0,6. Above the value of 0,6, the organoleptic properties of the dairy product may become less attractive. Most preferably, the said weight ratio is 0,2-0,5; within these limits, significant prebiotic effects can be obtained, without interfering with the organoleptic qualities of the dairy product.

In a preferred embodiment, the food product according to the invention comprises 0,5 w/w% or more α-lactalbumin, preferably 0,5-1,5 w/w%, more preferably about 1 w/w%. With "about" a deviation of 0,1 w/w% is allowed. A dairy product, especially milk and yoghurt, comprises 0,1-0,25% α-lactalbumin. By increasing the α-lactalbumin level according to the invention, the dairy product has a significant beneficial prebiotic effect.

Preferably, the protein part of the food product according to the invention comprises 10-40, preferably 20-35, most preferably 25-30 w/w% α-lactalbumin. α-Lactalbumin can be added to the dairy product or during the preparation thereof, in the form of e.g. whey protein concentrate (WPC) that is enriched in α-lactalbumin. The term "whey protein concentrate" is known in the art; see e.g. Walstra et al, 1999, Dairy Technology, ISBN 0-8247-0228-X. Such an enriched concentrate, herein also referred to as "αWPC", is known from EP-B-0 604 684. The α-lactalbumin content of αWPC may vary, depending on the preparation between 20-80 w/w%, whereas the α-lactalbumin content of normal WPC is about 12-18 w/w%. When a whey protein concentrate having an α-lactalbumin content below 20% is added to a dairy product, a relatively large amount thereof has to be added in order to obtain the desired α-lactalbumin level in the dairy product. In order to avoid possible negative organoleptic effects by adding a too high amount of protein to the dairy product, it is preferred to use protein concentrates, comprising at least 60 w/w% α-lactalbumin. With such concentrates, an α-lactalbumin content of 30 w/w% (on total protein basis) can be obtained in a dairy product, e.g. yoghurt, without any negative organoleptic effects

The food product according to the invention is preferably a dairy product. More preferably, the dairy product is a consumer product, even more preferably chosen from milk, fermented milk products, such as yoghurt, instant drinks, desserts, cheese and cheese products, most preferably being chosen from milk and fermented milk products, preferably yoghurt.

Further, the invention relates to a pharmaceutical composition for the treatment of gastroenteritis comprising α-lactalbumin as active compound. Herein, the term "gastroenteritis" is meant to encompass both gastritis and enteritis and simultaneous gastritis and enteritis in both acute and chronic forms.

As α-lactalbumin has been shown to be active as prebiotic agent, the said compound is very suitable for incorporation in a pharmaceutical composition against intestinal diseases, especially gastroenteritis. Any suitable administration form can be chosen for the pharmaceutical composition such as tablets, syrups, suppositories etc. For the preparation of the pharmaceutical composition, α-lactalbumin can be combined with any suitable carrier, diluent, excipient or any other suitable additives known in the art.

It has also been found that, in combination with a α-lactalbumin the presence of a biocompatible iron compound has a synergistic effect on the prebiotic activity, compared to that of α-lactalbumin in absence of such a compound; therefore the invention also relates to a food product or pharmaceutical composition comprising a food grade iron compound. The skilled person will be able to find the proper amount of such an iron containing compound to incorporate in the food product. Reference is made to Wharton et al., supra, herein incorporated by reference.

The invention will now be further discussed by the following examples and figures, wherein
Fig. 1 shows the effect of the presence of α-lactalbumin on the number of lactobacilli in rat faeces;
Figs. 2 and 3 show the effect of casein, WPC and α-lactalbumin on the amount of lactobacilli in the rat caecum and colon
Fig. 4 shows growth of lactobacilli in faeces extracts from rats, fed with diets containing different protein preparations;
Fig. 5 shows a graph of the effect of the α-lactalbumin content in the rat diet on excretion of *Salmonalla enteritidis;*

### EXAMPLES

### Example 1

### Effect of α-lactalbumin on the total lactobacillus counts in rat faeces

Male Wistar rats, SPF (specific pathogen free), six weeks old, were divided into groups as is depicted in Table 1. Animals were fed a diet containing 10% protein, 20% fat (50% palm oil, 50% corn oil), 63% glucose, 2% cellulose, and 5% of a vitamin and mineral mixture according to AIN 93. Water was supplied ad libidum.

**Table 1**

| Group | Rat Numbers | Protein source | g α-lac./100 g diet |
|---|---|---|---|
| A | 1, 4, 7, 10, 13, 16 | Casein | 0,00 |
| B | 2, 5, 8, 11, 14, 17 | WPC 80* | 0,48 |
| C | 3, 6, 9, 12, 15, 18 | αWPC** | 2,40 |

| | | | |
|---|---|---|---|
| * WPC 80 (Esprion 580, DMV International, The Netherlands). This product contains approximately 12% α-lactalbumin/protein | | | |
| ** α-lactalbumin enriched WPC 80 (60% α-lactalbumin/protein) | | | |

In the "casein" diet (A), the protein source was 100% casein.
In the "WPC" diet (B), the protein consisted of 50% casein and 50% WPC.
In the αWPC diet (C), the protein consisted of 50% casein and 50% α-WPC.

After 14 days, the amount of lactobacilli (LAB) was determined in the faeces. The total amount of lactobacilli is determined by plating dilutions of fresh faeces on MRS plates (see: J.C. de Man et al, Appl. Bact. 1960; 23: 130-135, purchased from E. Merck Nederland B.V., Amsterdam, The Netherlands). The composition of the lactobacilli flora was determined by analysing a representative sample of the colonies on the MRS plates by RAPD-PCR, followed by 16S RNA sequence analysis (H.J.M. Harmsen et al, J. Pediatr. Gastroenterol, Nutr. 2000; 30: 61-67).

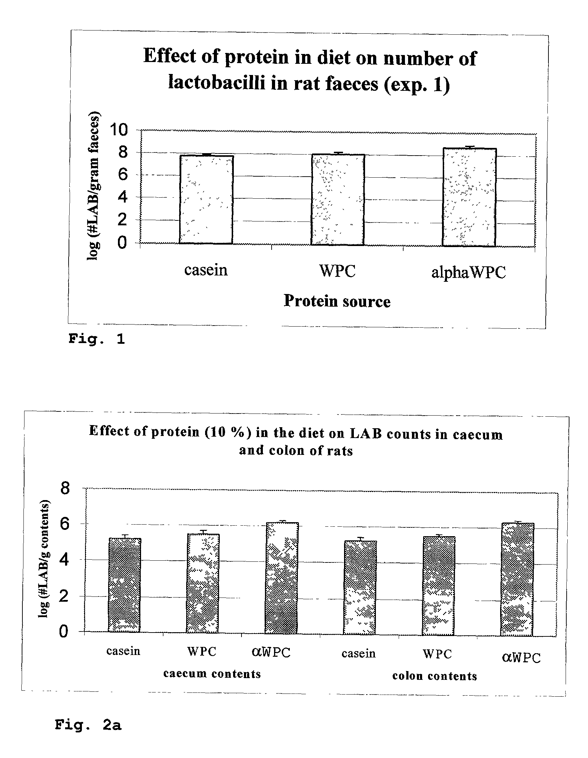

The results are shown in table 2 and in figure 1.

**Table 2.**

| Total numbers of lactobacilli in faeces of rats fed diets containing different kinds of protein. | | |
|---|---|---|
| Protein source | **Log10(lactobacilli/g faeces)** | SD |
| Casein | 7.76 | 0.14 |
| WPC | 8.01 | 0.20 |
| αWPC | 8.68 | 0.19 |

As is shown in table 2 and figure 1, the number of lactobacilli in rat faeces is significantly increased when feeding an αWPC diet, compared to a casein or WPC diet. α-Lactalbumin contains a high amount of cysteine, an important sulfur-containing amino acid. Without being bound to any explanation, it is believed that the prebiotic effect of αWPC may be due to one or more of the following aspects:
- Taurine is a breakdown product of cysteine and is, like glycine, a substrate for bile acid conjugation. Glycine-conjugated bile acids are more easily deconjugated than taurine-conjugated bile acids. Since deconjugated bile acids are toxic to lactic acid bacteria, a higher amount of cysteine in the diet could lead to more taurin-conjugated bile acids, which is in favor of the lactic acid bacteria population.
- Mucins are sulfur-containing substances, secreted by intestinal epithelial cells. When mucins no longer adhere to the intestinal epithelium, they are a substrate to lactic acid bacteria in the intestinal lumen. For this reason, a higher amount of cysteine in the diet could lead to increased mucin production, which favors the growth of lactic acid bacteria.
- Cysteine and glutathione, which is made from cysteine, are strongly reducing agents. A reducing environment promotes the survival of lactic acid bacteria.

### Example 2

### Effect of protein in the diet on the number of lactobacilli in the caecum and colon of the rat.

This experiment was performed as experiment 1. Rats were killed and the caecum was removed. Diets containing 10 or 20 % protein were used. A diet containing 20 % protein more closely resembles a human, western diet. In this case, part of the glucose in the diet was replaced by extra protein. In earlier experiments, only marginal diets containing 10 % protein were used to increase the chance of finding significant effects.

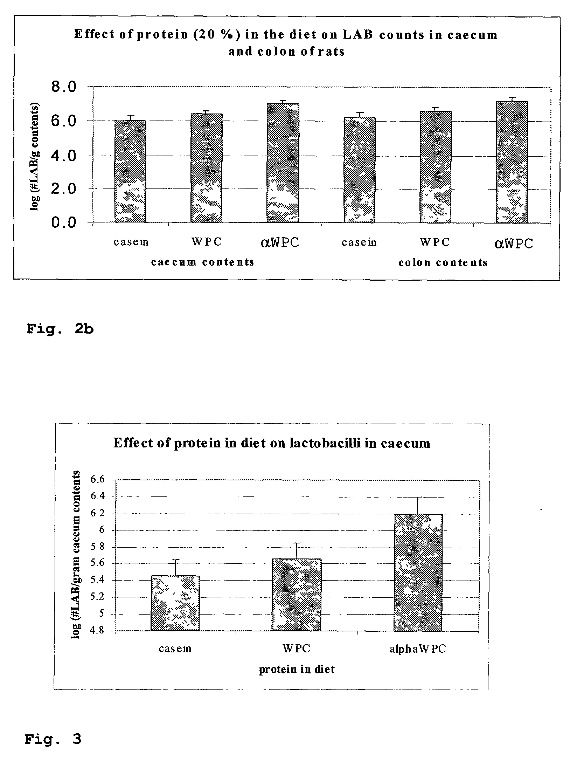

After the 14 day feeding period, the rats were killed and caecum and colon contents removed. Serial ten-fold dilutions of caecum and colon contents in physiological salt solution were plated onto MRS plates to determine the total *Lactobacillus* counts.
The results are shown in table 3 and figures 2A and 2B. The number of lactobacilli is significantly higher when feeding rats a diet containing αWPC compared to casein or WPC. The number of lactobacilli is significantly higher in both caecum and colon, and in both the 10 % and 20 % protein group when using αWPC instead of casein or WPC.

**Table 3**

| Effect of 10 and 20 % (w/w) of different kinds of protein in the rat diet on the number of lactobacilli in caecum and colon | | | | |
|---|---|---|---|---|
| **amount of protein** | **location** | **protein source** | **log(LAB/g)** | **SD** |
| 10% | caecum | casein | 5,2 | 0,2 |
| | | WPC | 5,5 | 0,2 |
| | | alphaWPC | 6,2 | 0,1 |
| | colon | casein | 5,2 | 0,2 |
| | | WPC | 5,5 | 0,1 |
| | | alphaWPC | 6,3 | 0,1 |
| 20% | caecum | casein | 6,0 | 0,3 |
| | | WPC | 6,4 | 0,2 |
| | | alphaWPC | 7,0 | 0,2 |
| | colon | casein | 6,2 | 0,3 |
| | | WPC | 6,6 | 0,2 |
| | | alphaWPC | 7,2 | 0,2 |

### Example 3

### Effect of protein in the rat diet on the total count and composition of the Lactobacillus population in the caecum.

The same rats as used in experiment 1 were used to determine the total number of lactobacilli in the caecum and to analyze the composition of the lactobacillus flora. At the end of the experiment, the rats were killed and the caecum was removed. The caecum content was diluted in a physiological salt solution by serial ten-fold dilution steps. Dilutions were plated onto MRS plates. The composition of the lactobacillus flora was determined by analyzing a representative sample of the colonies on the MRS plates by RAPD-PCR, followed by 16S RNA sequence analysis.

The results are shown in table 4 and figure 3. As shown in table 4 and figure 3, the number of lactobacilli in the rat caecum is significantly higher when using an αWPC diet as compared to a casein or WPC diet. Moreover, the lactobacillus flora is more diverse as well, which is generally accepted as beneficial. αWPC promotes the growth of *Lactobacillus reuteri* and *Lactobacillus acidophilus.*

**Table 4:**

| Effect of different kinds of protein in the rat diet on the number of lactobacilli and the composition of this flora in the caecum. | | | | | |
|---|---|---|---|---|---|
| **Protein source** | **# LAB in caecum (log cfu/g)** | **# rats containing <100% murinis** | ***L. murinis*** | ***L. reuteri*** | ***L. acidophilus*** |
| casein | 5.46 ± 0.19 | 2 (of 7) | 98 ± 1% | 2 ± 1% | 0 |
| WPC | 5.66 ± 0.19 | 2 (of 8) | 96 ± 3% | 4 ± 3% | 0 |
| αWPC | 6.20 ± 0.20 | 4 (of 8) | 87 ± 5% | 9 ± 5% | 4 ± 4% |

### Example 4

### Growth of lactobacilli in faecal extracts.

Growth of lactobacilli in 10 % (w/v) faecal extracts in water was monitored. The extracts were inoculated with *L. acidophilus* or *L. reuteri,* which were isolated from colonies obtained in experiment 3. By doing this, we used *Lactobacillus* species isolated from rats and characterized by 16S RNA analysis. The extracts, inoculated with lactobacilli, were incubated at 37 °C, under anaerobic conditions. Growth was monitored by plating serial ten-fold dilutions onto MRS plates.

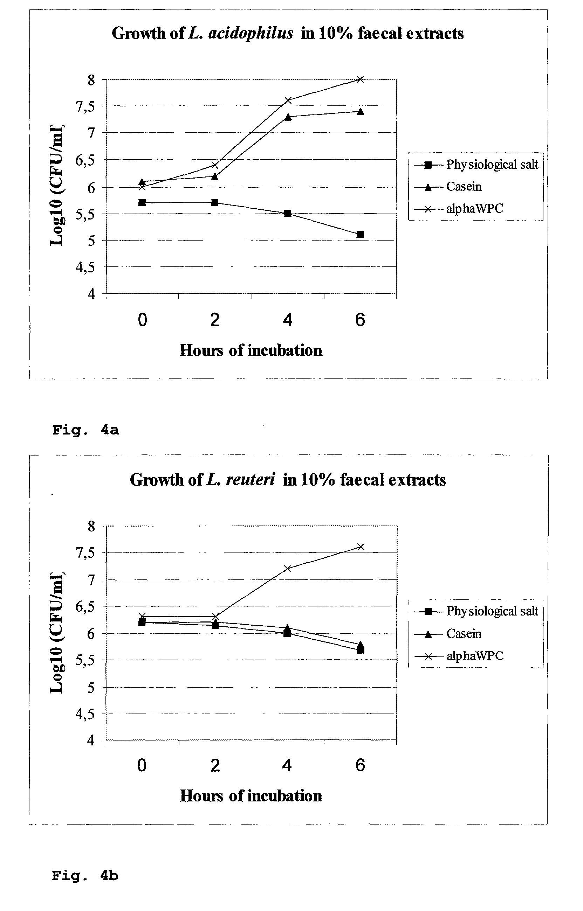

The results are shown in figures 4a and 4b. Figure 4a shows us that faecal extracts from rats consuming αWPC or casein diets support growth of *Lactobacillus acidophilus* to the same extent. *Lactobacillus reuteri,* however, is stimulated much more by faecal extracts from rats consuming αWPC as compared to casein. A diet rich in sulfur-containing amino acids (such as the αWPC diet) is expected to stimulate growth of lactobacilli, as explained earlier. In the case of growth in faecal extracts, the positive effect of increased mucin secretion on lactobacillus growth is believed to be the most profound. Mucins support growth of lactobacilli and can be detected in faeces. The found results correspond with the results of experiment 3, where αWPC also was found to stimulate *L. reuteri* and *L. acidophilus.*

### Example 5

### Effect of αWPC on the resistance against Salmonella

Male Wistar rats, SPF, six weeks old, were divided into groups as depicted in table 5.

**Table 5.**

| Experimental setup experiment 5. | | |
|---|---|---|
| Group | Rat numbers | Protein source |
| A | 1, 3, 5, 7, 9, 11, 13, 15 | Casein |
| B | 2, 4, 6, 8, 10, 12, 14, 16 | αWPC |

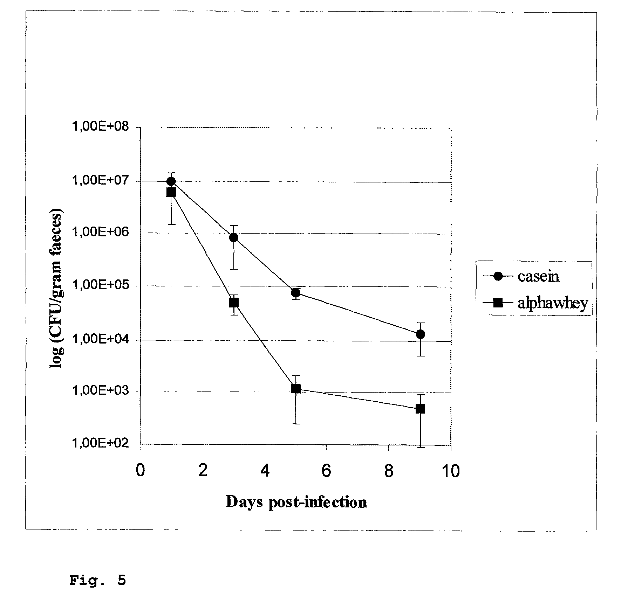

The rats were fed semi-synthetic diets, differing in protein source as indicated in table 5. After 14 days, the animals were orally given 1 ml of a suspension containing 5*10⁸ CFU *Salmonella enteritidis* in a physiological salt solution. The course of the infection was followed by plating dilutions of fresh faeces onto Rambach agar plates (see: A. Rambach, Appl. Environm. Microbiol., 1990; 56: 301-303, purchased from E. Merck Nederland B.V., Amsterdam, The Netherlands), a selective medium for *Salmonella* species.
The results are shown in figure 5. This figure indicates that using αWPC in the diet leads to a significantly quicker wash out of salmonella. A fast wash out of salmonella means a quick recovery from disease. In other words, when no more salmonella is excreted, the infection is over. As said and shown before, a diet rich in α-lactalbumin (so, rich in cysteine) will lead to increased numbers of lactic acid bacteria (LAB) in the gastro-intestinal tract. A strong LAB population will lead to increased colonization resistance to e.g. *Salmonella* because of competition for adherence sites and nutrients and because of a lowering of pH.

## Claims

1. Use of α-lactalbumin as a prebiotic food, food ingredient, food additive or food supplement.

2. Food product, comprising casein and α-lactalbumin in a ratio of 1:0,6-0,1, preferably of 1:0,5-0,2.

3. Food product, comprising 0,5 w/w% or more α-lactalbumin, preferably 0,5-1,5 w/w%, more preferably about 1 w/w%.

4. Food product, the protein part thereof comprising 10-40 w/w%, preferably 20-35 w/w%, most preferably 25-30 w/w% α-lactalbumin.

5. Food product according to any of the claims 2-4, being a dairy product, preferably chosen from milk, fermented milk products, desserts, instant drinks, cheese and cheese products, preferably chosen from milk and fermented milk products, preferably yoghurt.

6. Use of α-lactalbumin enriched whey protein concentrate as a prebiotic food, food ingredient, food additive or food supplement.

7. Pharmaceutical composition for the treatment of gastroenteritis comprising α-lactalbumin as active compound.

8. Pharmaceutical composition for the treatment of gastroenteritis comprising α-lactalbumin as active ingredient and casein, wherein the weight ratio α-lactalbumin:casein is 0,1 w/w% or more.

9. Pharmaceutical composition according to claim 8, wherein the ratio α-lactalbumin is 0,2 w/w% or more, preferably 0,4 w/w% or more, most preferably 0,6 w/w% or more.

10. Food product or pharmaceutical composition according to any of the claims 2-5, 7-9, comprising a food grade iron compound.
